(19) Europäisches Patentamt
European Patent Office
Office européen des brevets

(11) Publication number: **0 048 000 A2**

## (12) EUROPEAN PATENT APPLICATION

(21) Application number: 81107197.6

(22) Date of filing: 11.09.81

(51) Int. Cl.³: C 07 D 475/08
A 61 K 31/505

(30) Priority: 12.09.80 DK 3905/80

(43) Date of publication of application: 24.03.82 *Bulletin* 82/12

(84) Designated Contracting States: AT BE CH DE FR GB IT LI LU NL SE

(71) Applicant: Farmos Group Ltd.
Tengströminkatu 6 P.O. Box 425
SF-20101 Turku 10(FI)

(72) Inventor: Nedenskov, Poul
Lindebakken 36B
DK-3460 Birkerod(DK)

(72) Inventor: Jensen, Jens Karl
Berggreensgade 26
DK-2100 Copenhagen O.(DK)

(74) Representative: Patentanwälte Grünecker, Dr.Kinkeldey Dr.Stockmair, Dr.Schumann,Jakob, Dr.Bezold Meister, Hilgers, Dr.Meyer-Plath
Maximilianstrasse 43
D-8000 München 22(DE)

# (54) Method for preparing N-(4-(((2,4-diamino-6-pteridinyl)-methyl)methylamino)benzoyl)glutamic acid and analogues thereto.

(57) The invention relates to a method for preparing pteridine derivatives of the general formula I

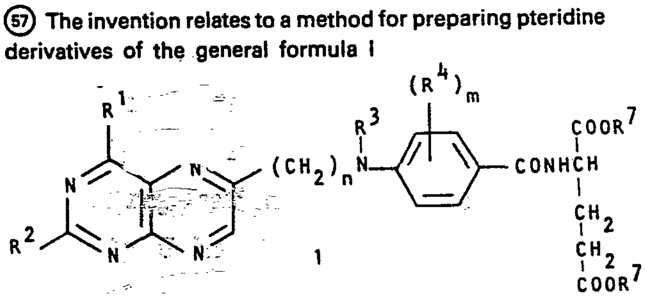

I

wherein R' is $NH_2$ or OH, $R^2$ is $NH_2$ or $NHR^5$, wherein $R^5$ is lower alkyl, $R^3$ is H or lower alkyl, m is an integer up to 4, n is an integer 0, 1, 2, 3, 4, $R^4$ is hydrogen, lower alkyl, halogen or trifluormethyl, and $R^7$ is H or lower alkyl, in L, D, or D,L-form, wherein a compound of the general formula II

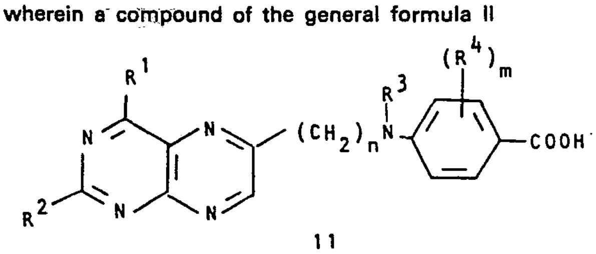

II

wherein $R^1$, $R^2$, $R^3$, $R^4$, m and n are as defined above, and, if required, $R^3$ in the meaning hydrogen is protected with a protecting group such as an acetyl or trifluoroacetyl group, is reacted with a compound of the general formula III

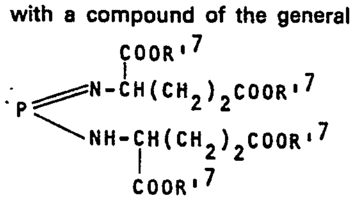

III

wherein $R'^7$ is lower alkyl, whereafter, if required, a protected amino group $NR^3$ is deprotected and $R'^7$ is, if desired, hydrolysed to yield a compound of formula I wherein $R^7$ is hydrogen.

The pteridine derivatives according to the present invention are antineoplastic agents useful in the treatment of acute leukemia and other cancerous diseases.

EP 0 048 000 A2

The present invention relates to a novel method for preparing pteridine derivatives, in particular methotrexate (N-[4-[[(2,4-diamino-6-pteridinyl)methyl]methyl-amino]benzoyl]glutamic acid) and analogues thereto.

Methotrexate has been extensively used for over 30 years in cancer chemotherapy as an antineoplastic agent useful in the treatment of acute leukemia and other cancerous diseases. In the past, methotrexate has been accepted in a rather impure state and with unspecified optical rotation (cf. U.S.P. XIX 1973-74, p. 315 and B.P. 73, p. 300).

Methotrexate may be represented by the formula

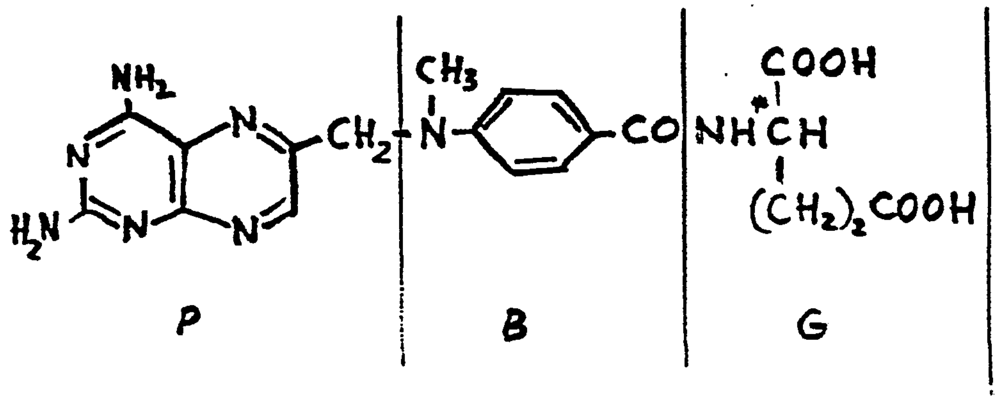

wherein the moieties of which the molecule is built up are represented as the "pteridine moiety", P, the "benzene moiety", B, and the "glutamate moiety", G. In all existing patent literature concerning the preparation of methotrexate and derivatives thereof, the glutamate moiety G has been attached to the benzene moiety B and then to molecules from which the pteridine moiety P was built up. However, the more steps in which the optically active G moiety participates, the higher is the risk of racemisation (cf. e.g. U.S. Patent No. 4,057,548 (1977) which describes a method where the final step to methotrexate diethylester involving building up the pyrimidine ring in the P-moiety results in complete racemisation).

N-acylation of L-glutamic acid where the PB moiety is transformed to an active acylating agent like a mixed anhydride and is then

used for acylation of L-glutamic acid is described in the literature (M. Chaykovsky et al., J. Med. Chem. (1975) 18, 909), but usually results in low yields (10 - 40%).

The present invention uses a completely new strategy in the synthesis of methotrexate and analogues thereto and provides an easy and clean-running procedure to obtain a high yield of methotrexate ester which may thereafter be hydrolysed to methotrexate.

In the method of the invention, the $NH_2$ group in the G moiety (as ester) is activated, and the activated G moiety is reacted with the PB moiety.

Hence, the invention provides a method of preparing compounds of the general formula I

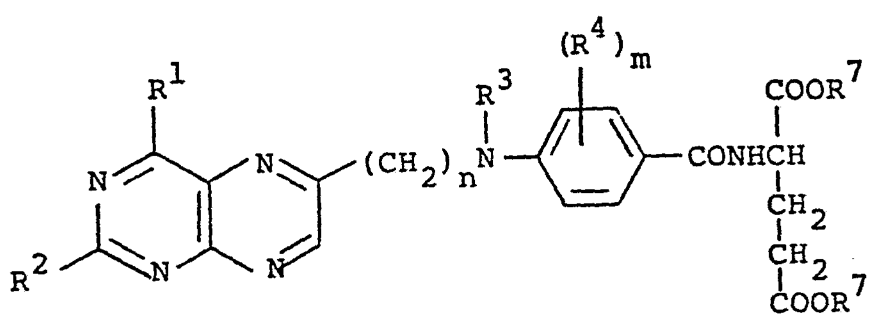

I

wherein $R^1$ is $NH_2$ or OH, $R^2$ is $NH_2$ or $NHR^5$, wherein $R^5$ is lower alkyl, $R^3$ is H or lower alkyl, m is an integer up to 4, n is an integer 0, 1, 2, 3, 4, $R^4$ is hydrogen, lower alkyl, halogen or trifluormethyl, and $R^7$ is H or lower alkyl, in L, D, or D,L-form, wherein a compound of the general formula II

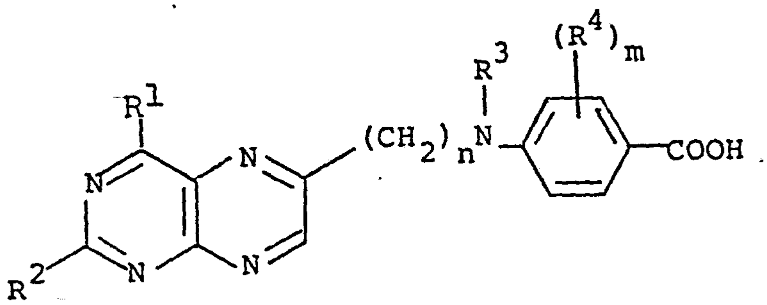

II

wherein $R^1$, $R^2$, $R^3$, $R^4$, m and n are as defined above, and, if required, $R^3$ in the meaning hydrogen is protected with a protecting group such as an acetyl or trifluoroacetyl group, is reacted with a compound of the general formula III

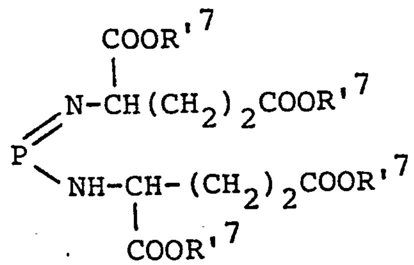

III

wherein $R'^7$ is lower alkyl, whereafter, if required, a protected amino group $NR^3$ is deprotected, and $R'^7$ is, if desired, hydrolysed to yield a compound of formula I wherein $R^7$ is hydrogen.

When $R^1$ and $R^2$ are each $NH_2$, $R^3$ is $CH_3$, and $R^4$ and $R^7$ are hydrogen, n = 1, and m = 4, the compound I is methotrexate, and a preferred embodiment of the invention comprises preparing methotrexate by reacting a corresponding compound II with a compound III wherein $R'^7$ is ethyl and thereafter hydrolysing resulting compound to yield methotrexate.

In the compound III, the glutamate moiety is preferably in optically active form such as L-form or D-form, and the resulting methotrexate will, correspondingly, be L- or D-methotrexate, respectively.

Compared with the conventional strategy for the synthesis of methotrexate, the process of the invention provides several advantageous features:

The moiety which is activated in the synthesis is the G moiety which is a much cheaper moiety than the GB moiety. This is an important advantage as the activated moiety is often used in excess. As mentioned above, no racemisation takes place in the synthesis stage. The PB moiety used in the preparation of methotrexate according to the strategy of the present invention may be made in a manner known *per se* (M. Chaykovsky et al., J. Med. Chem. 17 (1974), 1212), but according to one aspect of the invention, the preparation

of the PB moiety is modified to yield a particularly pure PB moiety, such as is discussed below in connection with the discussion of the preparation of the compounds of the general formula II.

The compounds III are novel compounds and constitute an aspect of the present invention. According to a preferred embodiment, $R'^7$ in the compounds III is ethyl. The compounds III are prepared by reacting phosphorus trichloride with a glutamic acid $R'^7$-diester. The phosphorus trichloride used in this reaction should be of a high purity. The reaction is suitably performed in an anhydrous basic reaction medium such as dry pyridine. The reaction product partially crystallises to form a crystal slurry which may be used in the reaction with compound II without further purification. This reaction is suitably performed in the same reaction medium.

As indicated above, the compounds of the general formula II in which $R^1$ and $R^2$ are $NH_2$, may be prepared as described in the literature or in analogy thereto (M. Chaykovsky et al., J. Med. Chem. 17 (1974), 1212), but preferably with special measures to reduce the content of the corresponding $N^{10}$-methylpteroic acid in order to increase the purity of the resulting compound of the general formula I. A suitable improved synthesis of the compounds II in which $R^1$ and $R^2$ are $NH_2$ comprises reacting a compound of the formula IV

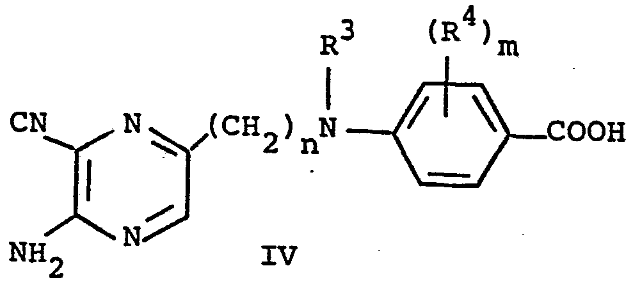

IV

wherein $R^3$, $R^4$, m and n are as defined above, with a salt of guanidine with a weak organic or inorganic acid such as a acetate or carbonate in a polar aprotic solvent such as dimethylsulfoxide. In a preferred embodiment of this process, the compound IV is 4-[[(5-amino-6-cyano-pyrazinyl)methyl]methylamino]benzoic acid which is reacted with

guanidine carbonate in dimethyl sulfoxide to yield 4-amino-4-deoxy-$N^{10}$-methylpteroic acid as the compound II.

The compounds of the general formula II in which $R^1$ is OH may be prepared in a corresponding manner, but using a compound of the general formula IV containing an ester group instead of the cyano group. Compounds of the general formula II in which $R^2$ is $NHR^5$ may be prepared in an analogous manner, using, instead of guanidine, an $R^2$-substituted guanidine.

S. Goldschmidt and Hans Lautenschläger, Ann. 581, 68 (1953), suggested the activation of $NH_2$ groups in amino acid esters by conversion into phosphorazo compounds for the synthesis of di- and tripeptides. However, this reference contains no indication of the use of phosphorazo compounds in an unorthodox new synthesis strategy for the preparation of methotrexate and analogues thereto.

Example 1.

A solution of $PCl_3$ (5.1 ml, 0.058 mol) in dry pyridine (20 ml) was added dropwise to a solution of diethyl L-glutamate hydrochloride (27.8 g, 0.116 mol) in dry pyridine (20 ml) with vigorous stirring at $0^oC$. After the addition, the thick crystal slurry was stirred for approximately 1 hour without cooling.

This slurry was poured into a slurry of 86% 4-amino-4-deoxy-$N^{10}$-methylpteroic acid (containing 9% $H_2O$ and 4.7% HCl) (8.7 g, 0.023 mol) in dry pyridine (150 ml) at $70^oC$. The resulting clear solution was kept at $110^oC$ for 2 hours whereafter it was cooled to $35^oC$ and filtered. The filtrate was concentrated under reduced pressure at 70 - $80^oC$. The residual oil was shaken with 250 ml $H_2O$, and the pH of the slurry formed was adjusted to 3 with N HCl (approx. 120 ml). The yellowish brown solid was filtered and washed with water. The filter cake was air dried, whereafter its weight was 12.8 g, corresponding to a quantitative yield of methotrexate diethylester hydrochloride.

A slurry of the hydrochloride in ethanol (600 ml) was neutralized to pH 9 with N NaOH (12 ml), whereafter filtration was performed. The filtrate was concentrated at 30$^{o}$C, and the residue was stirred with chloroform (500 ml) and water (400 ml). The chloroform phase was isolated, washed with a saturated sodium chloride solution and dried over anhydrous sodium sulfate. After filtration and evaporation of chloroform, the residue (10.4 g) was recrystallized from acetonitrile, to yield 7 g (60%) pure methotrexate diethylester, yielding, on saponification at room temperature in a mixture of ethanol (180 ml) and N sodium hydroxide (35 ml), methotrexate dihydrate in 89% yield. HPLC analysis of the product indicates a good purity of methotrexate when compared with an authentic sample.

Calculated for $C_{20}H_{22}N_8O_5$ + 8.8% $H_2O$ + 2.2% HCl:

| | | | | | |
|---|---|---|---|---|---|
| | C 47.0 | H 5.3 | N 21.9 | Cl 2.0 | $H_2O$ 8.8 |
| Found: | C 46.4 | H 5.4 | N 21.5 | Cl 2.0 | $H_2O$ 8.8 |

Optical rotation $[\alpha]^{D}_{23}$ = +19.9 ± 0.4° (c = 1 in 0.1N NaOH).

The IR (KBr) and PMR spectra were identical with those of an authentic sample.

The 4-amino-4-deoxy-$N^{10}$-methylpteroic acid used as starting compound was prepared as follows:

4-[[(5-amino-6-cyanopyrazinyl)methyl]methylamino]benzoic acid (e.g. prepared as described by Michael Chaykovsky et al. in J. Med. Chem. 1974, Vol. 17, No. 11, p. 1212 - 1216) (0.2 mol) was mixed with DMSO (500 ml) in a flask at room temperature. To the mixture was added guanidine carbonate (60 g, 0.66 mol) in one portion, and the resulting mixture was heated to 110$^{o}$C in 40 minutes and maintained at 110$^{o}$C for a further 80 minutes. Then the $NH_3$ evolution was very weak and the mixture was cooled and poured onto 2 liters of water at approx. 20$^{o}$C. A conventional acid base purification using charcoal in the filtrations gave a wet greasy residue (100 g) which was dried at room temperature overnight whereafter it was dried at 50$^{o}$C to constant weight. A yellowish powder (49 g) (65%) was obtained.

HPLC showed that the content of $N^{10}$-methylpteroic acid was below 1%.

Analysis.

Calculated for $C_{15}H_{25}N_7O_2$ (325.3), 4.6% HCl, 8.8% $H_2O$:

| | | | | | |
|---|---|---|---|---|---|
| | C 48.0 | H 5.2 | N 26.1 | Cl 4.6 | $H_2O$ 8.8 |
| Found: | C 47.6 | H 5.1 | N 25.5 | Cl 4.6 | $H_2O$ 8.8. |

Example 2.

N-[4-[[(2,4-Diamino-6-pteridinyl)methyl]methylamino]benzoyl]-D-glutamic acid (D-methotrexate).

The reaction in Example 1 was repeated with diethyl D-glutamate hydrochloride substituted for the L-isomer to give D-methotrexate $[\alpha]_{20}^{D}$ = -20 ± 1° (c = 1 in 0.1N NaOH).

The IR (KBr) and PMR spectra were identical with those of an authentic sample.

Example 3.

N-[4-[[(2,4-Diamino-6-pteridinyl)methyl]methylamino]benzoyl]-D,L-glutamic acid (D,L-methotrexate).

The reaction in Example 1 was repeated with diethyl D,L-glutamate hydrochloride substituted for the L-isomer to give D,L-methotrexate.

Calculated for $C_{20}H_{22}N_8O_5$ + 9.8% $H_2O$:

| | | | | |
|---|---|---|---|---|
| | C 47.7 | H 5.5 | N 22.2 | $H_2O$ 9.8 |
| Found: | C 47.6 | H 5.6 | N 22.0 | $H_2O$ 9.8 |

Optical rotation $[\alpha]_{25}^{D}$ = 0 ± 0.8° (c = 1 in 0.1N NaOH).

The IR (KBr) and PMR spectra were identical with those of an authentic sample.

Claims.

1. A method of preparing compounds of the general formula I

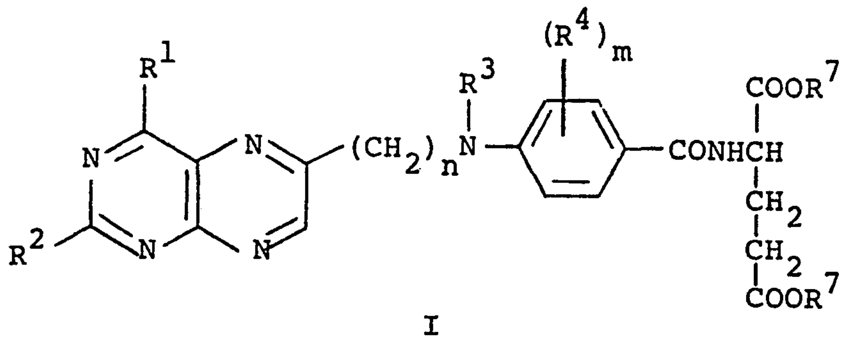

I

wherein $R^1$ is $NH_2$ or OH, $R^2$ is $NH_2$ or $NHR^5$, wherein $R^5$ is lower alkyl, $R^3$ is H or lower alkyl, m is an integer up to 4, n is an integer 0, 1, 2, 3, 4, $R^4$ is hydrogen, lower alkyl, halogen or trifluormethyl, and $R^7$ is H or lower alkyl, in L, D, or D,L-form, wherein a compound of the general formula II

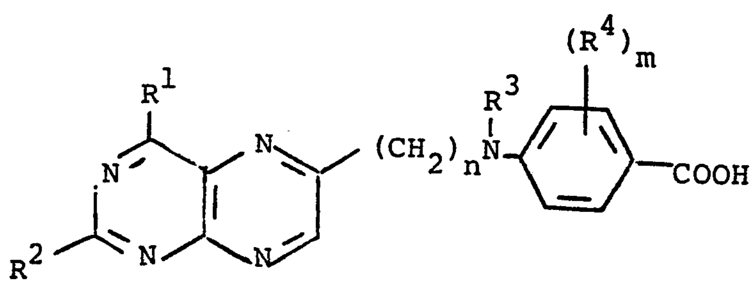

II

wherein $R^1$, $R^2$, $R^3$, $R^4$, m and n are as defined above, and, if required, $R^3$ in the meaning hydrogen is protected with a protecting group such as an acetyl or trifluoroacetyl group, is reacted with a compound of the general formula III

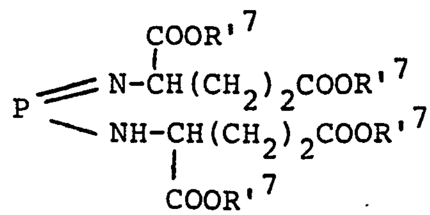

III

wherein $R'^7$ is lower alkyl, whereafter, if required, a protected

amino group $NR^3$ is deprotected, and $R'^7$ is, if desired, hydrolysed to yield a compound of formula I wherein $R^7$ is hydrogen.

2. A method as claimed in claim 1, wherein compounds of formula I, wherein $R^1$ and $R^2$ are $NH_2$, $R^3$ is methyl, n is 1, m is 4, $R^4$ is hydrogen, and $R^7$ is hydrogen, are prepared by reacting a corresponding compound of the general formula II with a compound of the general formula III, wherein $R'^7$ is ethyl, whereafter the ethyl group is hydrolysed off.

3. A method as claimed in claim 1 or 2, wherein the hydrolysis is performed using sodium hydroxide and aqueous ethanol.

4. A method as claimed in any of claims 1 - 3, wherein the compound prepared is the L-isomer.

5. A method as claimed in any of claims 1 - 3, wherein the compound prepared is the D-isomer.

6. A method as claimed in any of claims 1 - 3, wherein the compound prepared is the D,L-form.

7. Compounds of the general formula III

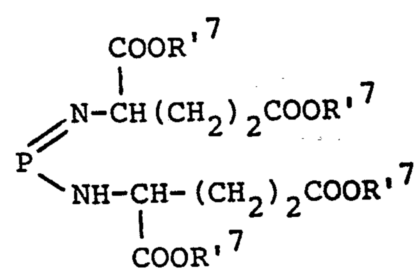

III

wherein $R'^7$ is as defined above.

8. A compound as claimed in claim 7 which is the L-isomer.

9. A compound as claimed in claim 7 which is the D-isomer.

10. A compound as claimed in claim 7 which is the D,L-form.

11. The compound of formula III'

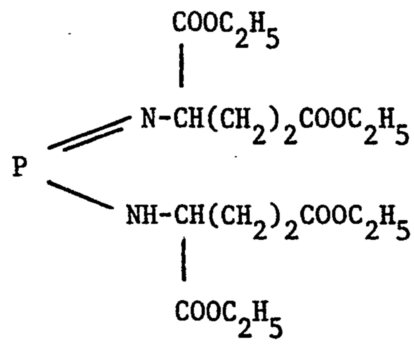

III'

12. A method of preparing a compound of formula III as defined in claim 7, wherein phosphorus trichloride is reacted with a glutamic acid diester.

13. A method as claimed in claim 12, wherein the glutamic acid diester is the L-isomer.

14. A method as claimed in claim 12, wherein the glutamic acid diester is the D-isomer.

15. A method as claimed in claim 12, wherein the glutamic acid diester is the D,L-form.

16. A method as claimed in any of claims 12 - 15, wherein the glutamic acid diester is glutamic acid diethyl ester.

17. A method as claimed in claim 1 in which $R^1$ and $R^2$ are $NH_2$, wherein the compound of formula II in which $R^1$ and $R^2$ are $NH_2$ is prepared by reacting a compound of the general formula IV

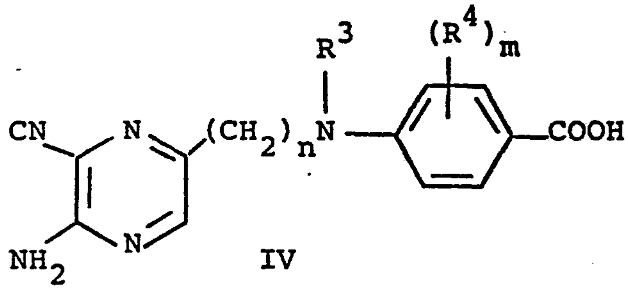

IV

wherein $R^3$, $R^4$, m and n are as defined in claim 1, with a salt of guanidine with a weak inorganic or organic acid, in a polar aprotic solvent.

18. A method as claimed in claim 17 wherein 4-[[(5-amino-6-cyano-pyrazinyl)methyl]methylamino]benzoic acid is reacted with guanidine carbonate in dimethyl sulfoxide to yield 4-amino-4-deoxy-$N^{10}$-methyl-pteroic acid.

19. Compounds of formula I, whenever prepared by a method as claimed in any of claims 1 - 6 or 17 - 18.

20. A method preparing a compound of formula II as defined in claim 1, in which $R^1$ and $R^2$ are $NH_2$, comprising reacting a compound of the general formula IV

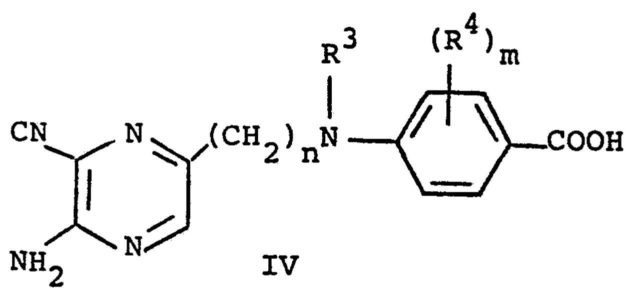

IV

wherein $R^3$, $R^4$, m and n are as defined in claim 1, with a salt of guanidine with a weak inorganic or organic acid, in a polar aprotic solvent.

21. A method as claimed in claim 20,wherein 4-[[(5-amino-6-cyano-pyrazinyl)methyl]methylamino]benzoic acid is reacted with guanidine carbonate in dimethyl sulfoxide to yield 4-amino-4-deoxy-$N^{10}$-methyl-pteroic acid.

22. Compounds of formula II, whenever prepared by a method as claimed in claim 20 or 21.